(19) Europäisches Patentamt
European Patent Office
Office européen des brevets

(11) **EP 1 492 511 B3**

(12) **NOUVEAU FASCICULE DE BREVET EUROPEEN**
Après la procédure de limitation (B3-1)

(45) Date de publication et mention de la décision de limitation:
**B3-1  02.05.2012   Bulletin 2012/18**

(45) Mention de la délivrance du brevet:
**07.01.2009   Bulletin 2009/02**

(21) Numéro de dépôt: **03745832.0**

(22) Date de dépôt: **07.04.2003**

(51) Int Cl.:
***A61K 9/50*** (2006.01)

(86) Numéro de dépôt international:
**PCT/FR2003/001096**

(87) Numéro de publication internationale:
**WO 2003/084518 (16.10.2003 Gazette 2003/42)**

(54) **FORMULATION PHARMACEUTIQUE ORALE SOUS FORME DE SUSPENSION AQUEUSE DE MICROCAPSULES PERMETTANT LA LIBERATION MODIFIEE DE PRINCIPE(S) ACTIF(S)**

ORALE WÄSSRIGE SUSPENSION, MIKROKAPSELN ENTHALTEND, ZUR KONTROLLIERTEN FREISETZUNG VON WIRKSTOFFEN

ORAL PHARMACEUTICAL FORMULATION IN THE FORM OF AQUEOUS SUSPENSION FOR MODIFIED RELEASE OF ACTIVE PRINCIPLE(S)

(84) Etats contractants désignés:
**AT BE BG CH CY CZ DE DK EE ES FI FR GB GR HU IE IT LI LU MC NL PT RO SE SI SK TR**

(30) Priorité: **09.04.2002  FR 0204409**
**02.09.2002  FR 0210847**

(43) Date de publication de la demande:
**05.01.2005   Bulletin 2005/01**

(73) Titulaire: **FLAMEL TECHNOLOGIES**
**69200 Vénissieux (FR)**

(72) Inventeurs:
• **CASTAN, Catherine**
**F-69530 Olienas (FR)**

• **GUIMBERTEAU, Florence**
**F-33450 Montussan (FR)**
• **MEYRUEIX, Rémi**
**F-69009 Lyon (FR)**

(74) Mandataire: **Fleurance, Raphaël et al**
**Cabinet Plasseraud**
**235 cours Lafayette**
**69006 Lyon (FR)**

(56) Documents cités:
**EP-A- 0 475 536     EP-A- 0 624 371**
**EP-A- 1 123 700     DE-A- 3 943 242**

**Description**

Le domaine de l'invention est celui de la libération modifiée de principes actifs pharmaceutiques, à l'exclusion de l'amoxicilline. Dans le présent exposé, l'expression "libération modifiée", désigne indifféremment une libération du (ou des) principe(s) actif(s) débutant dès la mise en contact de la forme galénique avec son milieu de dissolution (in vivo ou in vitro) ou bien encore une libération du (ou des) principe(s) actif(s) ne débutant qu'après une durée prédéterminée allant par exemple de 0,5 à plusieurs heures. Au sens de l'invention, le temps de libération de 50% du (ou des) principe (s) actif(s) est typiquement de plusieurs heures, et peut s'étendre de 0,5 à 30 heures, par exemple.

Plus précisément, l'invention concerne des formulations pharmaceutiques liquides, administrables oralement, à libération modifiée de principe(s) actif(s) à l'exclusion de l'amoxicilline. Ces formulations sont constituées par des suspensions ou dispersions de microcapsules formées chacune par un coeur comprenant au moins un principe actif (à l'exclusion de l'amoxicilline) et par un enrobage enveloppant ledit coeur. Les microcapsules constituant la phase dispersée de la suspension sont conçues, conformément à l'invention, pour permettre la libération modifiée du (ou des) principe(s) actif(s) à l'exclusion de l'amoxicilline.

Plus particulièrement encore, l'invention concerne notamment des suspensions aqueuses, "multimicrocapsulaires", de principe(s) actif(s) administrable(s) oralement, à l'exclusion de l'amoxicilline, lesquelles suspensions sont stables pendant toute la durée du traitement et permettent la libération modifiée du principe actif (à l'exclusion de l'amoxicilline). Ces suspensions sont particulièrement intéressantes dans le cas :

- des formes à libération modifiée de principes actifs ayant des doses thérapeutiques élevées (par exemple de 500 à 1000 milligrammes et plus) ;
- des formes pédiatriques ou gériatriques liquides à libération modifiée de principes actifs (comme par exemple les sachets ou les suspensions buvables à reconstituer en flacon) ;
- du masquage de goût et/ou de la protection des principes actifs fragiles.

L'invention vise également un procédé particulier de préparation des microcapsules destinées à être mises en suspension aqueuse.

Les formulations pharmaceutiques orales à libération modifiée de principe(s) actif(s), sont bien connues.

Certaines de ces formulations sont constituées par des comprimés comprenant un noyau thérapeutiquement actif revêtu avec des épaisseurs variées de matériaux non digestibles.

Plus récemment sont apparues des microcapsules ou des microsphères comportant un coeur de principe(s) actif(s) enrobé(s) dans des couches de perméabilité ou solubilité différentes. Ces microcapsules/microsphères sont placées par exemple dans des gélules de gélatine, de façon à former des systèmes galéniques à libération modifiée de principe(s) actif(s).

La plupart des formes pharmaceutiques à libération modifiée comprenant un noyau d'actif(s) enrobé, sont présentées sous forme solide : comprimés, gélules, microsphères, ou microcapsules.

A titre d'illustration de microcapsules sous forme sèche, on peut citer en particulier le brevet EP-B-0 709 087 qui décrit un système galénique (pharmaceutique ou diététique), de préférence sous forme de comprimé, avantageusement délitable, de poudre ou de gélule, caractérisé en ce qu'il comprend des microcapsules, de type réservoir, contenant au moins un Principe Actif médicamenteux et/ou nutritionnel (PA), notamment choisi parmi les antibiotiques, destinées à l'administration par voie orale, caractérisées :

- en ce qu'elles sont constituées par des particules de PA recouvertes chacune d'une pellicule d'enrobage de composition suivante :

    1-au moins un polymère filmogène (P1) insoluble dans les liquides du tractus, présent à raison de 50 à 90 % en poids sur sec par rapport à la masse totale de la composition d'enrobage et constitué par au moins un dérivé non hydrosoluble de la cellulose, éthylcellulose et/ou l'acétate de cellulose étant particulièrement préférés ;
    2-au moins un polymère azoté (P2) présent à raison de 2 à 25% en poids sur sec par rapport à la masse totale de la composition d'enrobage et constitué par au moins un polyacrylamide et/ou un poly-N-vinylamide et/ou un poly-N-vinyl-lactame, le polyacrylamide et/ou la polyvinylpyrrolidone étant particulièrement préféré(s) ;
    3-au moins un plastifiant présent à raison de 2 à 20 % en poids sur sec par rapport à la masse totale de la composition , d'enrobage et constitué par au moins l'un des composés suivants : les esters du glycérol, les phtalates, les citrates, les sébacates, les esters de l'alcool cétylique, l'huile de ricin, l'huile de ricin étant particulièrement préférée ;
    4-au moins un agent tensio-actif et/ou lubrifiant, présent à raison de 2 à 20 % en poids sur sec par rapport à la masse totale de la composition d'enrobage et choisi parmi les tensio-actifs anioniques, de préférence les sels alcalins ou alcalinoterreux des acides gras, l'acide stéarique et/ou oléique étant préféré(s) et/ou parmi les tensio-actifs non ioniques, de préférence les esters de sorbitan polyoxyéthylénés et/ou les dérivés de l'huile de ricin polyoxyéthylénés, et/ou parmi les agents lubrifiants comme les stéarates, de préférence de calcium, de magnésium, d'aluminium ou de zinc, ou comme le stéarylfumarate, de préférence de sodium, et/ou le béhénate de glycérol ; ledit agent pouvant comprendre un seul ou un mélange des susdits produits ;

- en ce qu'elles possèdent une granulométrie comprise entre 50 et 1 000 microns ;
- en ce qu'elles sont conçues de manière à pouvoir séjourner dans l'intestin grêle pendant un temps d'au moins 5 heures environ, et permettre ainsi l'absorption du PA pendant au moins une partie de leur temps de séjour dans l'intestin grêle.

Ce document ne concerne que les formes pharmaceutiques sèches à base de microcapsules et ne mentionne nullement les formes pharmaceutiques liquides orales à base de microcapsules.

Ces formulations pharmaceutiques solides à libération modifiée ne sont pas toujours avantageuses, notamment lorsqu'elles sont administrées à dé très jeunes enfants ou à des patients très âgés ayant des difficultés à avaler.

Il en est ainsi dans le cas où les principes actifs concernés doivent être administrés oralement à doses élevées, par exemple de 500 à 1000 milligrammes et plus, comme c'est le cas par exemple pour la metformine. Il est clair que de tels systèmes galéniques solides sont inadaptés car trop volumineux et donc très difficiles à avaler, notamment par les jeunes enfants ou les personnes âgées. Cela peut générer une mauvaise observance par les patients et par la suite une mise en péril du succès du traitement thérapeutique.

De même dans le cas des formes pédiatriques où la dose thérapeutique doit être adaptée en fonction de la masse de l'enfant, les suspensions de l'invention sont adaptées aux flacons pourvus de seringues graduées en kg déjà existants et ne nécessitent donc pas la mise au point d'un dispositif nouveau. Les formes à libération modifiée jusqu'ici peu employées pour les enfants sont donc désormais accessibles grâce à l'invention. Les avantages de telles formes sont la réduction du nombre de prise quotidienne et l'optimisation de l'efficacité du traitement entre chaque prise (comme par exemple pour les antibiotiques, les anti-inflammatoires, les traitements cardiovasculaires, ...). Ainsi, une formulation pharmaceutique liquide à libération contrôlée, qui soit facile à préparer, constituerait un progrès significatif.

Dans ce cas, il serait encore plus avantageux de mettre en oeuvre des systèmes galéniques à libération modifiée, constitués d'une pluralité de microcapsules de diamètre inférieur à 1000 microns. En effet, dans ces systèmes, la dose de principe(s) actif(s) à administrer se répartit entre un grand nombre de microcapsules (typiquement 10 000 pour une dose de 500 mg) et présente, de ce fait, les avantages intrinsèques suivants :

- La mise en oeuvre d'un mélange de microcapsules de profils de libération modifiée différents, permet de réaliser des profils de libération présentant plusieurs vagues de libération ou assurant, par un réglage adéquat des différentes fractions, un niveau de concentration plasmatique du PA constant.
- On évite la mise en contact des tissus avec une dose élevée en PA « dose dumping ». Chaque microcapsule ne contient en effet qu'une dose très réduite en principe(s) actif(s). On s'affiranchit ainsi du risque de détérioration des tissus par surconcentration locale de principe(s) actif(s) agressif(s).
- Il est possible de combiner plusieurs formes galéniques (libération immédiate ou modifiée) comportant un ou plusieurs principe(s) actif(s) dans ces systèmes "multimicrocapsulaires".

On connaît des formes galéniques multiparticulaires liquides, plus précisément des suspensions colloïdales, qui sont préférées aux formes solides pour les principes actifs fortement dosés ou pour les applications pédiatriques, administrables par voie orale.

La réalisation de suspensions liquides à libération modifiée de principe(s) actif(s) est délicate. La difficulté principale à résoudre est d'éviter la libération du (ou des) principe(s) actif(s) dans la phase liquide durant le stockage de la suspension, tout en permettant une libération modifiée dès son entrée dans le tractus gastro-intestinal. Cet objectif est particulièrement délicat à atteindre puisque le (ou les) principe(s) actif(s) est (sont) conservé(s) dans un liquide pendant une très longue durée au regard du temps de libération souhaité dans les fluides du tractus gastro-intestinal. Par ailleurs, son séjour prolongé dans la phase liquide de stockage ne doit pas perturber le système à libération modifiée au point d'entraîner une altération du profil et du temps de libération du (ou des) principe(s) actif(s).

Par ailleurs, pour que ces formulations liquides soient pleinement efficaces, on sait qu'il importe :

o que les microcapsules soient de très petite traille (< 1000 microns),
o et que la fraction massique en excipients d'enrobage soit limitée, cette modalité étant d'autant plus délicate à obtenir que, du fait de leur faible taille, les microcapsules ont une grande surface spécifique, ce qui accélère la libération.

S'agissant de l'art antérieur sur les formes pharmaceutiques liquides, orales à libération modifiée de principes actifs, il convient de mentionner tout d'abord la demande de brevet français FR-A-2 634 377 qui divulgue une nouvelle forme pharmaceutique à libération modifiée à base d'un complexe résine/principe actif, enrobé par un polymère ionique de polarité opposée à celle de la résine et fixé à elle par liaison ionique. Le principe actif est également ionique et possède une polarité opposée à celle de la résine. Cette dernière peut être du polystyrène sulfonate de sodium et le polymère ionique enrobant est choisi parmi les polymères d'ester d'acide acrylique et méthacrylique (EUDRAGIT®). La résine est imprégnée par une solution aqueuse du principe actif. Les particules de résine imprégnées de principe actif sont ensuite enrobées par une solution organique de polymère ionique. Les microcapsules ainsi obtenues peuvent être mises sous forme de suspension buvable (notamment exemple 2). L'utilisation de résine et de polymère d'enrobage ioniques limite les possibilités d'application aux principes actifs ioniques.

Les brevets américains US-B-4,999,189 et US-B-5,186,930 concernent des compositions pharmaceutiques liquides comprenant des complexes *résine ionique échangeuse d'ions /principe actif* en suspension dans une phase liquide. Ces particules de complexe *résine/ principe actif* sont enrobées par une première couche de cire pharmaceutiquement acceptable et à haut point de fusion et par une seconde couche extérieure d'un polymère insoluble dans l'eau et pharmaceutiquement acceptable (éthylcellulose ou méthylcellulose hydroxypropylméthylcellulose, hydroxyéthyl-cellulose, Eudragit®...). Un plastifiant tel que le dibutylsébacate peut être introduit dans cette seconde couche extérieure. Le principe actif est fixé par liaison ionique sur la résine échangeuse d'ions. La phase liquide est constituée par un sirop de glucose à forte teneur en fructose et par plusieurs autres ingrédients tels que la glycérine ou le propylène glycol.

Le brevet US-B-5,186,930 se distingue du premier en ce qu'il prévoit une quantité de première couche (cire) suffisante pour empêcher le gonflement et le craquèlement des particules de complexes résines/principes actifs.

Ces brevets US ne fournissent aucun élément permettant d'apprécier la qualité de la libération modifiée de principes actifs. En outre, le fait d'employer des résines échangeuses d'ions comme support du principe actif est peu commode et limitant en termes de variété de principes actifs concernés. De plus, ce système galénique ne démontre rien de convaincant au regard de la stabilité et de la préservation des propriétés de libération modifiée du principe actif.

La demande de brevet PCT WO-A-87/07833 et le brevet US-B-4,902,513 divulguent des suspensions aqueuses de microcapsules de principe actif (e.g. théophylline), à libération modifiée du principe actif (12h e.g.). Ces suspensions sont préparées en saturant la phase aqueuse avec le principe actif, avant d'incorporer les microcapsules de principe actif dans cette phase aqueuse. La composition de l'enrobant des microcapsules, permettant la libération modifiée du principe actif, n'est pas décrite dans ces documents. Or, cette composition d'enrobage est déterminante pour garantir le maintien du profil de libération modifiée des microcapsules, après conservation dans la phase aqueuse. Il apparaît que la proposition technique décrite ne divulgue pas les moyens permettant de résoudre le double problème de la réalisation d'une suspension liquide d'une forme microcapsulaire à libération modifiée, sans porter atteinte à la stabilité du profil de libération modifiée du principe actif à l'issue du stockage des microcapsules en phase liquide.

La demande de brevet européen EP-A-0 601 508 concerne une suspension aqueuse pour l'administration orale de naproxène, selon un profil de libération modifiée. Cette suspension comprend des microgranules de naproxéne enrobés, en suspension dans une phase liquide aqueuse sirupeuse. Le problème technique sous-tendant cette invention est la fourniture d'une forme à libération modifiée de naproxène, dosée à 1000 mg et administrable en une seule prise quotidienne.

Les microgranules sont constitués de naproxène, de polyvinylpyrrolidone, et de lactose (90-300 $\mu$m). Leur enrobage est fait de 4 couches. La première comprend: diéthylcellulose/diéthylphtalate/polyéthylèneglycol. La deuxième est à base de copolymères (meth)acrylates/(meth)acryliques EUDRAGIT. La troisième comporte du stéarate de glycérol/de la cire/des alcools gras. Et la quatrième est constituée par un revêtement entérique à base d'acétate/phtalate de cellulose. La libération de naproxène intervient de manière modifiée sur 24 heures.

L'exemple 22 de cette demande de brevet européen EP-A-0601508 comporte une démonstration de la stabilité du profil de libération après 30 jours de stockage de la suspension liquide.

L'un des inconvénients de cette suspension résulte de la couche entérique qui interdit l'usage d'une suspension à pH neutre car cette couche est conçue pour se déliter et devenir liquide à ce pH neutre. Un autre inconvénient de cette couche entérique est qu'elle bloque la libération du principe actif dans l'estomac, à pH acide. Or, pour les PA dont la fenêtre d'absorption est située dans les parties hautes du tractus gastrointestinal, il est souvent avantageux de libérer le principe actif dans l'estomac, pour augmenter la biodisponibilité. Par ailleurs, il s'agit d'une solution multicouches très complexe et de surcroît spécifique au naproxène.

La demande de brevet PCT WO-A-96/01628 divulgue une formulation pharmaceutique liquide pour l'administration orale, selon un profil de libération modifiée (12 heures), d'un principe actif constitué par de la moguistéine. Le but visé est de proposer une formulation liquide de moguistéine à libération modifiée, qui soit facile à mesurer et à ingérer, qui ait une durée de libération permettant d'éviter les multiples prises, qui soit stable en suspension aqueuse dans le temps, qui soit savoureuse pour favoriser le respect de l'observance et dont la fabrication n'implique pas le recours à des matières toxiques telles que des solvants. Pour atteindre ce but, l'invention selon la demande de brevet PCT WO-A-96/01628 propose une suspension dans une phase liquide faiblement hydratée (essentiellement à base de sorbitol et de glycérine), de microgranules (90-300 $\mu$m) de moguistéine enrobés par une première couche hydrophile constituée d'acétate/phtalate de cellulose et de diéthylphtalate; par une seconde couche hydrophobe comportant du stéarate de glycérol/de la cire/des alcools gras; et par une troisième couche hydrophile identique à la première.
Cette forme multicouches est très complexe à préparer et, de surcroît, est spécifique à la moguistéine.

Dans cet état de la technique, la présente invention a pour objectif essentiel de proposer une suspension aqueuse, de microcapsules de principe(s) actif(s) à l'exclusion de l'amoxicilline, pour l'administration orale de ce (ou ces) dernier(s) selon un profil de libération modifiée, dans laquelle enrobage des microcapsules est conçu de telle sorte que le profil de libération n'est pas perturbé et ne dépend pas du temps de macération des microcapsules dans la phase liquide (de préférence aqueuse) . Ainsi, serait évitée la libération du (ou des) principe(s) actif(s) contenu(s) dans les microcapsules, dans la phase liquide tout au long du stockage de la suspension, tout en permettant une libération modifiée du (ou des) principe(s) actif(s) dès son entrée dans un environnement permettant le déclenchement de la libération, à savoir in vivo dans le tractus gastro-intestinal et in vitro dans les conditions d'un test de dissolution réalisé juste après la mise en suspension des microcapsules dans la phase solvant (de préférence aqueuse), à l'aide d'un appareil de type II, selon la pharmacopée européenne 3ème édition, dans un milieu tampon phosphate pH 6,8, pour un volume de 900 ml, à une température de 37°C.

Un autre objectif de la présente invention est de fournir une suspension liquide aqueuse de microcapsules de principe(s) actif(s) (à l'exclusion de l'amoxicilline) comprenant une pellicule d'enrobage formée par une seule couche.

Un autre objectif de la présente invention est de fournir une suspension liquide aqueuse de microcapsules de principe(s) actif(s) (à l'exclusion de l'amoxicilline), dans laquelle la fraction de PA dissoute, issue des microcapsules, est inférieure ou égale à 15 %, de préférence à 5 % en poids de la masse totale de principes actifs présents dans les microcapsules.

Un autre objectif de la présente invention est de fournir une suspension liquide aqueuse de microcapsules de principe(s) actif(s) (à l'exclusion de l'amoxicilline) dans laquelle une partie du (ou des) principe(s) actif(s) est sous forme à libération immédiate et l'autre partie du (ou des) principe(s) actif(s) est sous forme à libération modifiée (microcapsules).

Un autre objectif essentiel de la présente invention est de fournir une suspension aqueuse de microcapsules. à libération modifiée de principe(s) actif(s) (à l'exclusion de l'amoxicilline) permettant de libérer le principe actif selon un profil de libération qui n'est pas altéré par le temps de vieillissement de la suspension.

Un autre objectif essentiel de la présente invention est de fournir une suspension aqueuse de microcapsules faite de particules de principe(s) actif(s) (à l'exclusion de l'amoxicilline) enrobées individuellement, et permettant la libération de ce (ou ces) dernier(s) selon un profil prolongé et/ou éventuellement retardé, tel que le temps de demi-libération $t_{1/2}$ soit compris entre 0,5 et 30 heures.

Un autre objectif de la présente invention est de proposer une forme galénique orale qui soit liquide et constituée d'un grand nombre (par exemple de l'ordre de plusieurs milliers) de microcapsules, cette multiplicité assurant statistiquement une bonne reproductibilité de la cinétique de transit du PA dans tout le tractus gastro-intestinal, de sorte qu'il en résulte un meilleur contrôle de la biodisponibilité et donc une meilleure efficacité.

Un objectif essentiel de la présente invention est de proposer une forme galénique liquide, orale et formée d'une pluralité de microcapsules enrobées évitant l'emploi de fortes quantités d'enrobant, la fraction massique d'enrobant étant comparable à celle des formes monolithiques.

Un autre objectif essentiel de la présente invention est de fournir une suspension aqueuse à libération modifiée dans laquelle le (ou les) principe(s) actif(s) (à l'exclusion de l'amoxicilline) est (sont) sous forme de pluralité de particules enrobées individuellement pour former des microcapsules et permettant le mélange de plusieurs principes actifs, libérés selon des temps de libération respectifs différents.

Un autre objectif essentiel de la présente invention est de proposer l'utilisation, à titre de moyen de traitement de maladies humaines ou vétérinaires, d'une suspension (de préférence aqueuse) de microcapsules constituées par des particules de principe(s) actif(s) (à l'exclusion de l'amoxicilline) enrobées individuellement de façon à déterminer la libération modifiée du (ou des) principe(s) actif(s), sans que le stockage sous cette forme liquide des microcapsules dans la suspension n'ait d'incidence sur le profil de libération modifiée.

Un autre objectif essentiel de la présente invention est de proposer un médicament à base d'une suspension (de préférence aqueuse) de microcapsules constituées par des particules de principe(s) actif(s) (à l'exclusion de l'amoxicilline) enrobées individuellement de façon à déterminer la libération modifiée du (ou des) principe(s) actif(s), sans que le stockage sous cette forme liquide des microcapsules dans la suspension n'ait d'incidence sur le profil de libération modifiée.

S'étant fixé tous les objectifs ci-dessus parmi d'autres, les inventeurs ont eu le mérite de mettre au point un système galénique multimicrocapsulaire sous forme de suspension, de préférence aqueuse, à libération modifiée de principe(s) actif(s) à l'exclusion de l'amoxicilline:

- qui n'altère pas le profil de libération modifiée, éventuellement retardée,
- et qui soit stable, facile à préparer, économique et performante.

Pour ce faire, les inventeurs ont proposé :

- de sélectionner une composition d'enrobage tout à fait particulière pour les microcapsules,
- et de mettre les microcapsules en suspension dans une phase liquide (de préférence aqueuse) saturée en principe (s) actif(s) ou saturable en principe(s) actif(s) au contact des microcapsules, tout en mettant en oeuvre une quantité limitée de solvant (de préférence l'eau), mais néanmoins suffisante pour que la suspension puisse être aisément avalée.

Ainsi, l'invention qui satisfait aux objectifs exposés ci-dessus, parmi d'autres, concerne une suspension de microcapsules dans une phase liquide aqueuse, permettant la libération modifiée d'au moins un principe actif (à l'exclusion de l'amoxicilline) et destinée à être administrée par voie orale, caractérisée :

- en ce qu'elle comprend une pluralité de microcapsules constituées chacune par un coeur comportant au moins un principe actif (à l'exclusion de l'amoxicilline) et par une pellicule d'enrobage:

  • appliquée sur le coeur,
  • régissant la libération modifiée du (ou des) principe(s) actif(s),
  • et ayant une composition correspondant à l'une des familles A et C suivantes :

    ⇒ Famille A

    - 1A -au moins un polymère filmogène (P1) insoluble dans les liquides du tractus, présent à raison de 50 à 90%, de préférence 50 à 80% en poids sur sec par rapport à la masse totale de la composition d'enrobage et constitué par au moins un dérivé non hydrosoluble de la cellulose;
    - 2A -au moins un polymère azoté (P2) présent à raison de 2 à 25, de préférence 5 à 15 % en poids sur sec par rapport à la masse totale de la composition d'enrobage et constitué par au moins un polyacrylamide et/ou un poly-N--vinylamide et/ou un polyN-vinyl-lactame;

    - 3A -au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin;
    - 4A -au moins un agent tensio-actif et/ou lubrifiant, présent à raison de 2 à 20, de préférence de 4 à 15 % en poids sur sec par rapport à la masse totale de la composition d'enrobage et choisi parmi les tensio-actifs anioniques, et/ou parmi les tensio-actifs non ioniques, et/ou parmi les agents lubrifiants; ledit agent pouvant comprendre un seul ou un mélange des susdits produits;

    ⇒ Famille C :

    - 1C -au moins un polymère filmogène insoluble dans les liquides du tractus gastro-intestinal,

    - 2C -au moins un polymère hydrosoluble,

    - 3C -au moins un plastifiant,

    - 4C -et éventuellement au moins un agent tensioactif/lubrifiant de préférence sélectionné dans le groupe de produits suivants:

      - les tensioactifs anioniques,
      - et/ou les tensioactifs non ioniques;

- et en ce que la phase liquide est saturée ou se sature en principe(s) actif(s) au contact des microcapsules.

Au sens du présent exposé, le terme "*microcapsules* de principe(s) actif(s)" désigne des microcapsules dont le coeur comprend du (ou des) principe(s) actif(s) et éventuellement au moins un excipient.

Cette suspension selon l'invention permet de surmonter les deux principaux obstacles à la réalisation d'une suspension aqueuse de microcapsules constituées par des microparticules de principes actifs enrobées individuellement et permettant la libération modifiée de ce dernier, ces deux obstacles étant de :

a) limiter la fraction de principes actifs libérables immédiatement à partir des microcapsules, à une valeur inférieure à 15 %, et de préférence à 5 % en poids de la masse totale de principes actifs mis en oeuvre dans les microcapsules,
b) obtenir un système à libération modifiée suffisamment robuste pour éviter toute évolution ou altération du profil de libération du (ou des) principe(s) actif(s) durant la durée de stockage de la suspension aqueuse.

En outre, cette suspension permet de faciliter l'administration orale de médicaments dont les doses thérapeutiques sont élevées et ce, notamment vis-à-vis des personnes âgées et des enfants, le gain en termes d'observance et de succès du traitement étant significativement élevé.

Par ailleurs, pour des PA dont la fenêtre d'absorption est limitée, il est particulièrement avantageux que la forme à libération modifiée soit une pluralité de microcapsules, comme cela est indiqué dans le préambule du présent exposé.

Selon une forme de réalisation préférée de l'invention, les familles A et C dans lesquelles sont choisies les constituants de la composition d'enrobage, sont les suivantes :

⇒ Famille A

- 1A - éthylcellulose et/ou acétate de cellulose;
- 2A - polyacrylamide eUou polyvinyl-pyrrolidone;
- 3A - huile de ricin;
- 4A - sel alcalin ou alcalinoterreux des acides gras, l'acide stéarique et/ou oléique étant préférés, ester de sorbitan polyoxyéthyléné, dérivé de l'huile de ricin polyoxyéthylène, stéarate, de préférence de calcium, de magnésium, d'aluminium ou de zinc, stéarylfumarate, de préférence de sodium, béhénate de glycérol ; pris à eux seuls ou en mélange entre eux ;

⇒ Famille C :

- 1C

~ les dérivés non hydrosolubles de la cellulose, l'éthylcellulose et/ou l'acétate de cellulose étant particulièrement préférés,
~ les dérivés acryliques,
~ les polyvinylacétates,
~ et leurs mélanges.

- 2C

~ les dérivés hydrosolubles de la cellulose,
~ les polyacrylamides,
~ les poly-N-vinylamides,
~ les poly-N-vinyl-lactames,
~ les alcools polyvinyliques (APV),
~ les polyoxyéthylénes (POE),
~ les polyvinylpyrrolidones (PVP) (ces dernières étant préférées),
~ et leurs mélanges ;

- 3C

~ le glycérol et ses esters, de préférence dans le sous-groupe suivant: glycérides acétylés, glycérolmonostéarate, glycéryl-acétate, glycéroltributyrate,
~ les phtalates, de préférence dans le sous-groupe suivant : dibutylphtalate, dléthylphtalate, diméthylphtalate, dioctylphtalate,
~ les citrates, de préférence dans le sous-groupe suivant : acétyltributylcitrate, acétyltriéthylcitrate, tributylcitrate, triéthylcitrate,
~ les sébacates, de préférence dans le sous-groupe suivant : ~ diéthylsébacate, dibutylsébacate.
~ les adipates,
~ les azélates,
~ les benzoates,
~ les huiles végétales,

~ les fumarates, de préférence le diéthylfumarate,
~ les malates, de préférence le diéthylmalate,
~ les oxalates, de préférence le diéthyloxalate,
~ les succinates, de préférence le dibutylsuccinate,
~ les butyrates,
~ les esters de l'alcool cétylique,
~ les malonates, de préférence le diéthylmalonate,
~ l'huile de ricin (celle-ci étant particulièrement préférée),
~ et leurs mélanges;

• 4C

~ les sels alcalins ou alcalinoterreux des acides gras, l'acide stéarique et/ou oléique étant préféré(s),
~ les huiles polyoxyéthylénées, de préférence l'huile de ricin hydrogénée polyoxyéthylénée,
~ les copolymères polyoxyéthylène-polyoxypropylène,
~ les esters de sorbitan polyoxyéthylénés,
~ les dérivés de l'huile de ricin polyoxyéthylénés,
~ les stéarates, de préférence de calcium, de magnésium, d'aluminium ou de zinc,
~ les stéarylfumarates, de préférence de sodium,
~ le béhénate de glycérol,
~ et leurs mélanges.

De préférence, la pellicule d'enrobage est constituée par une seule couche, dont la masse représente de 1 à 50 % en poids, de préférence de 5 à 40 % en poids, de la masse totale des microcapsules.

Suivant une caractéristique préférée de l'invention, la phase liquide est aqueuse, et plus préférentiellement encore, elle est constituée d'au moins 20% d'eau, et mieux encore d'au moins 50 % en poids d'eau.

Cette suspension selon l'invention comporte, avantageusement :

- 30 à 95 % en poids, de préférence 60 à 85 % en poids de phase liquide (avantageusement d'eau),
- 5 à 70 % en poids de préférence 15 à 40 % en poids de microcapsules.

Avantageusement, la quantité de phase liquide solvant (de préférence l'eau) du ou (des) principe(s) actif(s) (à l'exclusion de l'amoxicilline) est telle que la proportion de principe(s) actif(s) dissous et provenant des microcapsules soit inférieure ou égale à 15 %, de préférence à 5 % en poids par rapport à la masse totale de principe(s) actif(s) contenu dans les microcapsules.

Selon un premier mode de réalisation de l'invention, la phase liquide est au moins en partie, de préférence totalement, saturée en principe(s) actif(s) (à l'exclusion de l'amoxicilline) consécutivement à l'incorporation des microcapsules dans cette phase liquide.

Selon ce mode de réalisation, la saturation en principe(s) actif(s) s'opère au moyen du principe(s) actif(s) contenu(s) dans les microcapsules.

Selon un deuxième mode de réalisation de l'invention, la phase liquide est au moins en partie, de préférence totalement, saturée en principe(s) actif(s) (à l'exclusion de l'amoxicilline) à l'aide de principe(s) actif(s) non encapsulé, avant l'incorporation des microcapsules dans cette phase liquide. Ce mode de réalisation est particulièrement intéressant pour l'administration de principe actif (à l'exclusion d'amoxicilline), en ce qu'il permet d'associer une fraction à libération immédiate et une fraction à libération modifiée.

En pratique, cela revient à saturer la phase liquide en principe(s) actif(s) avant l'introduction des microcapsules dans la suspension, de sorte que le principe actif contenu dans les microcapsules ne participe pas, ou quasiment pas, à la saturation de la phase liquide. La diffusion du principe actif contenu dans les microcapsules est donc supprimée ou quasiment supprimée.

Suivant une caractéristique préférée de l'invention permettant à cette formulation orale liquide d'être pleinement efficace, les microcapsules ont une granulométrie inférieure ou égale à 1000 microns, de préférence comprise entre 200 et 800 microns, et, plus préférentiellement encore, entre 200 et 600 microns. Par « granulométrie », on entend au sens de l'invention, une proportion d'au moins 75 % en poids de microcapsules de diamètre compris entre les limites considérées de taille de grille de tamisage.

Toujours dans le souci d'améliorer l'efficacité, la quantité d'enrobant des microcapsules représente avantageusement de 1 à 50 %, de préférence 5 à 40 %, du poids des microcapsules enrobées. Cette caractéristique avantageuse est d'autant plus délicate à acquérir que les microcapsules, du fait de leur faible taille, ont une grande surface spécifique, ce qui accélère la libération.

Pour contrôler la libération in vivo in vitro du (ou des) principe(s) actif(s), il est préférable, conformément à l'invention, de mettre en oeuvre une pellicule d'enrobage pour les microcapsules, qui relève de la famille A ou C .

Pour plus de données détaillées sur le plan qualitatif et quantitatif, pour ce qui concerne cette composition d'enrobage de famille A, on se référera au brevets européen EP-B-0 709 087 dont le contenu est intégré dans le présent exposé par référence.

Un autre moyen de définition de la suspension liquide selon l'invention peut consister à faire état d'un profil de libération in vitro réalisé à l'aide d'un appareil de type II, selon la pharmacopée européenne 3ème édition, dans un milieu tampon phosphate pH 6, 8, et à une température de 37°C, tel que:

la proportion (%) PI de principe(s) actif(s) libéré(s) des microcapsules durant les 15 premières minutes du test de dissolution est telle que :

$$PI \leq 15$$

de préférence

$$PI \leq 5,$$

la libération du (ou des) principe(s) actif(s) restant(s) dans les microcapsules s'effectue sur une durée telle que le temps de libération de 50 % poids de PA ($t_{1/2}$) se définit comme suit (en heures) :

$$0,5 \quad \leq t_{1/2} \leq 30$$

de préférence

$$0,5 \quad \leq t_{1/2} \leq 20.$$

Toujours en ce qui concerne ses propriétés de dissolution in vitro, la suspension selon l'invention est caractérisée en ce que :

- le profil de libération in vitro initial Pfi, réalisé juste après la mise en suspension des microcapsules dans la phase solvant (de préférence aqueuse), à l'aide d'un appareil de type II, selon la pharmacopée européenne 3ème édition, dans un milieu tampon phosphate pH 6,8, pour un volume de 900 ml, à une température de 37°C,
- le profil de libération in vitro Pf$_{10}$, réalisé 10 jours après la mise en suspension des microcapsules dans la phase solvant (de préférence aqueuse), à l'aide d'un appareil de type II, selon la pharmacopée européenne 3ème édition, dans un milieu tampon phosphate pH 6,8, à une température de 37°C,

sont similaires.

Les profils de libération comparés selon les recommandations de l'Agence Européenne pour l'Evaluation des produits médicinaux -Unité d'évaluation des médicaments humains- "The European Agency for the Evaluation of Medicinal Products (EMEA)- Human Medicines Evaluation Unit- / Committee for proprietary medicinal products (CPMP) - London, 29 july 1999 *CPMP/QWP/604/96 : note for guidance on quality of modified release products : A :oral dosage forms B : transdermal dosage forms -section I (quality)- Annex 3 : Similarity factor f$_2$*", conduisent à une valeur des facteurs de similarité f$_2$ >50 et peuvent donc être déclarés similaires.

Ces caractéristiques avantageuses de la suspension selon invention permettent d'administrer oralement, de façon aisée, des doses élevées de principe(s) actif(s) sans nuire au mode de libération modifiée et éventuellement retardée du principe actif.

Suivant une autre de ses caractéristiques physico-chimiques avantageuses, le pH de la suspension liquide selon l'invention peut être indifféremment acide ou neutre.

Il peut être tout à fait intéressant d'adjoindre à la suspension au moins un agent modificateur de la rhéologie. Il peut s'agir en particulier d'un ou plusieurs agent(s) "Viscosifiant(s)" choisi(s) parmi ceux communément employés dans l'industrie pharmaceutique et notamment ceux divulgués dans "Handbook of pharmaceutical excipients - 3 rd Edition, Am. Pharmaceutical Association, Arthur H KIBBE, 2000, ISBN 0917330-96-X. *Europe.* 0-85369-381-1". A titre d'exemples, on peut citer:

- les dérivés hydrosolubles de la cellulose (hydroxyéthyl cellulose, hydroxypropyl cellulose, carboxyméthyl cellulose, hydroxypropylméthyl cellulose ...) ;
- les polyéthylèneglycols ;
- les alginates et leurs dérivés;
- les carraghénanes ;
- l'agar-agar ;
- la gélatine ;
- les maltodextrines ;
- le polydextrose ;
- les gommes guar, caroube, acacia, xanthane, gellane, ...
- l'alcool polyvinylique ;
- la povidone ;
- les pectines ;
- le gel de silice ;
- les amidons natifs, modifiés et leurs dérivés ;
- les dextrans.
- etc ...

Il peut être également judicieux d'introduire dans la suspension au moins un agent modificateur de la solubilité du principe actif dans la phase liquide solvant (de préférence aqueuse), comme par exemple des sels, des sucres, du glycérol, .... En effet, dans le cas des principes actifs très solubles, ces solutés peuvent limiter la sortie du principe actif des microcapsules par abaissement de la concentration à saturation du principe actif en phase aqueuse.

Pour donner à la suspension toutes les qualités d'une forme galénique orale, facile à avaler, stable et appétente, il est avantageux qu'elle comprenne au moins un autre additif choisi dans le groupe comprenant : les tensioactifs, les colorants, les agents dispersants, les conservateurs, les agents de sapidité, les arômes, les édulcorants, les anti-oxydants et leurs mélanges.

A titre d'exemples de ces additifs, on peut citer ceux communément employés dans l'industrie pharmaceutique et notamment ceux divulgués dans "Handbook of pharmaceutical excipients - 3 rd Edition, Am. Pharmaceutical Association, Arthur H KIBBE, 2000, ISBN 0917330-96-X. Europe. 0- 85369-381-1" ou s'agissant des émulsifiants, ceux décrits à la page 5, ligne 14 à 29 de l'EP-A- 0 273 890, ou bien encore s'agissant des épaississants, ceux indiqués à la page 5 lignes 19 et 20 de l'EP-A-0 601 508.

Les principes actifs utilisés pour la préparation des suspensions à libération contrôlée selon l'invention peuvent être choisis parmi au moins l'une des grandes variétés de substances actives suivantes :

antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens. neuroleptiques, hypnotiques, anxiolytiques, psychostimulants, antimigraineux, antidépresseurs, antitussifs, antihistaminiques ou antiallergiques,

Sans que cela ne soit limitatif, l'invention s'applique plus particulièrement aux principes actifs pharmaceutiques qui doivent être administrés oralement, à doses élevées, par exemple de 500 à 1000 milligrammes ou plus et aux suspensions pédiatriques.

De préférence, le ou les principe(s) actif(s) est (sont) choisi(s) parmi les composés suivants : pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril. simvastatine, fluoxétine. alprazolam, famotidine, ganciclovir, famciclovir, spironolactone, 5-asa, quinidine, périndopril, morphine, pentazocine, metformine, paracétamol, oméprazole, métoctopramide, aténolol, salbutamol morphine, vérapamil, erythromycine, caféine, furosémide, céphalosporines, montelukast, valacyclovir, sels d'acide ascorbique, diazépam, théophilline, ciprofloxacine, vancomycine, aminoglycosides, pénicillines (à l'exception de l'amoxicilline) et leurs mélanges.

Selon un autre de ses aspects, la présente invention concerne un médicament caractérisé en ce qu'il comprend de la suspension de microcapsules de principe(s) actif(s) à libération modifiée, telle que définie ci-dessus.

Plus concrètement, l'invention vise également un médicament, ou plus exactement un conditionnement galénique, caractérisé en ce qu'il comprend un nécessaire de préparation de la suspension telle que définie ci-dessus, lequel nécessaire comportant :

- des microcapsules sous forme sensiblement sèche contenant le (ou les) principe(s) actif(s) pour permettre la saturation de la phase liquide en principe(s) actif(s), une fois réalisée la mise en présence des deux phases solide et liquide;
- et/ou un mélange de microcapsules sous forme sensiblement sèche comprenant la dose en principe(s) actif(s) juste nécessaire pour la libération modifiée ainsi qu'une quantité nécessaire et suffisante de principe(s) actif(s) non enrobé(s) à libération immédiate, pour permettre la saturation de la phase liquide en principe(s) actif(s), une fois réalisée la mise en présence de la dose de saturation de principe(s) actif(s) et de la phase liquide;
- et la phase liquide et/ou au moins une partie des ingrédients utiles à sa préparation et/ou le protocole de préparation de la suspension.

Ce type de présentation du médicament selon l'invention permet aux patients de préparer aisément la suspension à libération modifiée, sous une forme stable, en particulier en termes de libération modifiée, et ce pendant au moins plusieurs jours. Le patient est donc ainsi garanti de disposer d'un médicament facilement administrable oralement, et parfaitement efficace sur le plan thérapeutique.

S'agissant de la préparation des microcapsules constituant la phase solide de la suspension selon l'invention, cela renvoie à des techniques de microencapsulation accessibles à l'homme du métier, dont les principales sont résumées dans l'article de C. DUVERNEY et J.P. BENOIT dans "L'actualité chimique", décembre 1986. Plus précisément, la technique considérée est la microencapsulation par pelliculage, conduisant à des systèmes "réservoir individualisés par opposition aux systèmes matriciels.

Pour plus de détails, on se référera au brevet EP-B-0 953 359 mentionné ci-avant.

Pour la réalisation du coeur à base de principe(s) actif(s) (à l'exclusion de l'amoxicilline) des microcapsules selon l'invention, il est avantageux d'utiliser comme matières premières des particules de principe(s) actif(s) de granulométrie désirée. Ces dernières peuvent être des cristaux de principe(s) actif(s) purs et/ou ayant subi un prétraitement par l'une des techniques conventionnelles en la matière, comme par exemple la granulation, en présence d'une faible quantité d'au moins un agent liant classique et/ou d'un agent modifiant les caractéristiques de solubilité intrinsèque du PA.

L'invention sera mieux comprise sur le plan de sa composition de ses propriétés et de son obtention à la lecture des exemples ci-après, donnés uniquement à titre d'illustration et permettant de faire ressortir les variantes de réalisation et les avantages de l'invention.

Description des figures :

- **La figure 1** montre les profils de dissolution initial et après 10 jours de conservation de la suspension selon l'exemple 1, en % dissous (D) en fonction du temps (t) en heures.
- **La figure 2** montre les profils de dissolution initial et après 19 jours de conservation de la suspension selon l'exemple 2, en % dissous (D) en fonction du temps (t) en heures
- **La figure 3** montre les profils de dissolution initial et après 12 jours de conservation de la suspension selon l'exemple 3, en % dissous (D) en fonction du temps (t) en heures. Cette suspension associe 29% de metformine libre et 71% de metformine encapsulée.

Exemple 1

*Préparation de microcapsules d'acyclovir :*

1000 g d'acyclovir et 30 g de povidone® sont mélangés à sec pendant 5 minutes. Le mélange est granulé à l'eau. Les granulés sont séchés à 40°C en étuve ventilée, puis calibrés sur grille de 500 μm. On sélectionne la fraction 200-500 μm.

700 g de granulés obtenus précédemment sont enrobés par 27,3 g d'éthylcellulose, 3,7 g d'huile de ricin, 3,7 g de stéarate de magnésium et 2,9 g de povidone® dissous dans un mélange acétone / isopropanol 60/40 m/m, dans un appareil à lit d'air fluidisé Glatt GPC-G1. Température produit : 40°C.

*Préparation de la suspension :*

0,58 g de microcapsules obtenues ci-dessus sont placés dans 37 ml de tampon phosphate pH 6,8.

*Test :*

La suspension ci dessus est conservée pendant 10 jours à température ambiante. Au bout de 10 jours, la suspension est analysée en dissolution. Appareil de type II selon la pharmacopée Européenne 3è édition, milieu tampon phosphate pH 6,8, volume 900 ml, température 37°C, agitation palettes 100 tours/min, détection UV 252 nm.

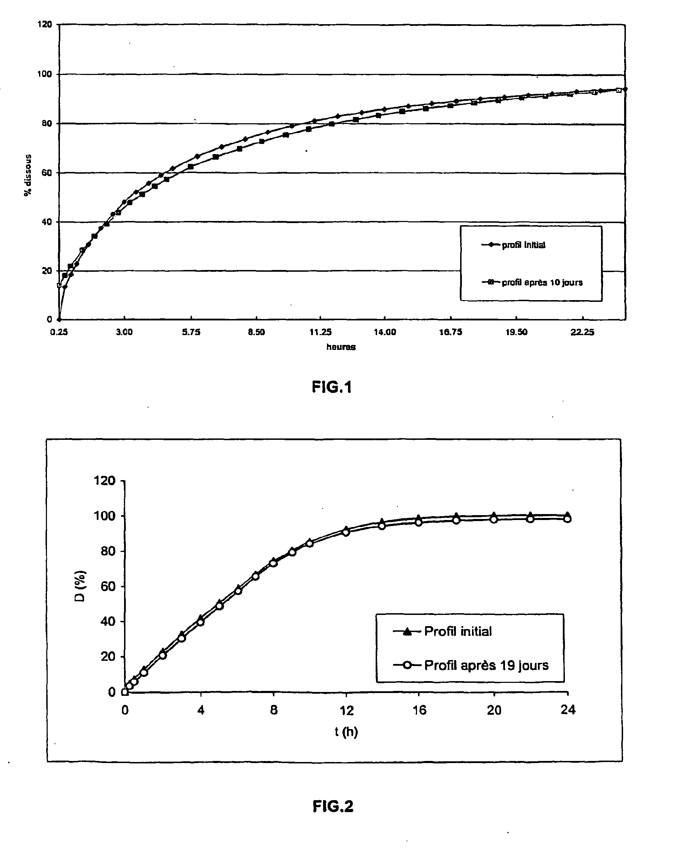

Le résultat est représenté sur la figure 1 annexée.

Il apparaît que les profils sont identiques : facteur de similarité $f_2$ supérieur à 50. Les microcapsules restent bien performantes en suspension aqueuse.

Exemple 2

*Préparation de microcapsules de spironolactone :*

*Etape 1 : Granulé*

45 g de spironolactone, 25 g de PEG 40-huile de ricin hydrogénée et 30 g de Povidone sont préalablement solubilisés dans un mélange eau / acétone / isopropanol (5/57/38 m/m). Cette solution est ensuite pulvérisée sur 800 g de sphères de cellulose (de diamètre compris entre 300 et 500 $\mu$m) dans un appareil à lit d'air fluidisé Glatt GPC-G1.

*Etape 2 : Enrobage*

50 g de granulés obtenus précédemment sont enrobés par 1,44 g d'éthylcellulose, 0,16 g d'huile de ricin, 0,64 g de poloxamer 188 et 0,96 g de povidone dissous dans un mélange acétone / isopropanol (60140 m/m), dans un appareil à lit d'air fluidisé miniGlatt.

*Préparation de la suspension :*

0,07 g de microcapsules obtenues ci-dessus sont placés dans 0,165 ml de tampon phosphate pH 6,8.

*Test:*

La suspension ci dessus est conservée pendant 19 jours à température ambiante. Au bout de 19 jours, la suspension est analysée en dissolution. Appareil de type II selon la pharmacopée Européenne 3è édition, milieu tampon phosphate pH 6,8, volume 1000 ml, température 37°C, agitation palettes 100 tours/min, détection UV 240 nm.

Le résultat est représenté sur la figure 2 annexée.

Il apparaît que les profils sont identiques : facteur de similarité $f_2$ supérieur à 50. Les microcapsules restent bien performantes en suspension aqueuse.

Exemple 3

*Préparation de microcapsules de metformine:*

740 g de cristaux de metformine (fraction 200-500 $\mu$m) sont enrobés par 192,4 g d'éthylcellulose, 26 g d'huile de ricin, 26 g de stéarate de magnésium et 20,8 g de povidone® dissous dans un mélange acétone / isopropanol 60/40 m/m, dans un appareil à lit d'air fluidisé Glatt GPC-G1. Température produit : 40°C.

Préparation de la suspension (29% forme libre et 71 % forme encapsulée):

50 g de microcapsules obtenues ci-dessus sont mélangées à sec avec 15 g de cristaux de metformine et 0,7 g de gomme xanthane dans un flacon en verre de 100 ml. 34,3 g d'eau purifiée sont ensuite ajoutés sur le mélange de poudre.

Après agitation manuelle, une suspension qui sédimente très lentement est obtenue.

Le titre en metformine totale dans la suspension est de 0,52 g/ml.

*Test de stabilité:*

La suspension ci dessus est conservée pendant 12 Jours à température ambiante. Au bout de 12 jours, la suspension est analysée en dissolution. Appareil de type **II** selon la pharmacopée Européenne 3è édition, milieu tampon phosphate pH 6,8, volume 900 ml, température 37°C, agitation palettes 100 tours/min, détection UV 232 nm.

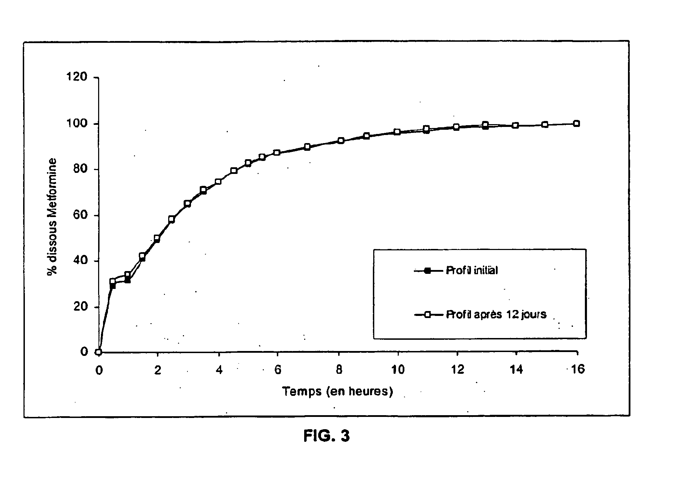

Le résultat est représenté sur la figure 3 annexée.

Il apparaît que les profils sont identiques : facteur de similarité $f_2$ supérieur à 50. Les microcapsules restent bien performantes en suspension aqueuse.

*Test d'homogénéité :*

La suspension ci-dessus est agitée manuellement puis à l'aide, d'une seringue graduée, 6 échantillons de 5 ml sont prélevés. La teneur en metformine de chaque prélèvement est déterminée par HPLC et reportée ci-dessous :

| Prélèvement n° | Teneur en metformine pour 5 ml de suspension (en g) |
|:---:|:---:|
| 1 | 2,58 |
| 2 | 2,60 |
| 3 | 2,62 |
| 4 | 2,59 |
| 5 | 2,60 |
| 6 | 2,63 |

On constate que les prélèvements sont bien homogènes et que le dosage correspond à la valeur attendue de 2,60 g pour 5 ml.

Cette formulations peut donc être administrée sans risque de sur- ou sous-dosage.

## Revendications

**1.** Suspension de microcapsules dans une phase liquide aqueuse, permettant la libération modifiée d'au moins un principe actif à l'exclusion de l'amoxicilline et destinée à être administrée par voie orale, **caractérisée :**

en ce qu'**elle comprend une pluralité de microcapsules constituées chacune par un coeur comportant au moins un principe actif (à l'exclusion de l'amoxicilline) et par une pellicule d'enrobage:

• appliquée sur le coeur,
• régissant la libération modifiée du (ou des) principe(s) actif(s),
• et ayant une composition correspondant à l'une des familles A et C suivantes :

⇒ Famille A

♦ 1A -au moins un polymère filmogène (P1) insoluble dans les liquides du tractus gastro-intestinal, présent à raison de 50 à 90%, de préférence 50 à 80 % en poids sur sec par rapport à la masse totale de la composition d'enrobage et constitué par au moins un dérivé non hydrosoluble de la cellulose;
♦ 2A -au moins un polymère azoté (P2) présent à raison de 2 à 25% de préférence 5 à 15 % en poids sur sec par rapport à la masse totale de la composition d'enrobage et constitué par au moins un polyacrylamide et/ou un poly-N-vinylamide et/ou un poly-N-vinyllactame;
♦ 3A -au moins un plastifiant présent à raison de 2 à 20%, de préférence de 4 à 15 % en poids sur sec par rapport à la masse totale de la composition d'enrobage et constitué par au moins l'un des composés suivants : les esters du glycérol, les phtatates, les citrates, les sébacates, les esters de l'alcool cétylique, l'huile de ricin;
♦ 4A -au moins un agent tensio-actif et/ou lubrifiant, présent à raison de 2 à 20%, de préférence de 4 à 15 % en poids sur sec par rapport à la masse totale de la composition d'enrobage et choisi parmi les tensio-actifs anioniques, et/ou parmi les tensio-actifs non ioniques, et/ou parmi les agents lubrifiants; ledit agent pouvant comprendre un seul ou un mélange des susdits produits;

⇒ Famille C :

♦ 1C -au moins un polymère filmogène insoluble dans les liquides du tractus gastro-intestinal,
♦ 2C -au moins un polymère hydrosoluble,
♦ 3C -au moins un plastifiant,
♦ 4C -et éventuellement au moins un agent tensioactif/lubrifiant de préférence sélectionné dans le groupe de produits suivants:

- les tensioactifs anioniques,

- et/ou les tensioactifs non ioniques; ■ en ce qu'elle comporte :

- 30 à 95 % en poids, de préférence 60 à 85 % en poids de phase liquide (avantageusement d'eau),
- 5 à 70 % en poids de préférence 15 à 40 % en poids de microcapsules ;

■ en ce que la quantité de phase liquide solvant (de préférence l'eau) du (ou des) principe(s) actif(s) est telle que la proportion de principe(s) actif(s) dissous et provenant des microcapsules soit inférieure ou égale à 15 %, de préférence à 5 % en poids par rapport à la masse totale de principe(s) actif(s) contenu(s) dans les microcapsules;
■ et en ce que la phase liquide est saturée ou se sature en principe(s) actif(s) au contact des microcapsules.

2. Suspension selon la revendication 1, **caractérisée en ce que** les familles A et C dans lesquelles sont choisis les constituants de la composition d'enrobage sont les suivantes :

■ <u>Famille A :</u>

♦ 1A - éthylcellulose et/ou acétate de cellulose;
♦ 2A - polyacrylamide et/ou polyvinyl-pyrrolidone;
♦ 3A - huile de ricin;
♦ 4A - sels alcalins ou alcalinoterreux des acides gras, l'acide stéarique et/ou oléique étant préféré(s), ester de sorbitan polyoxyéthyléné, dérivé de l'huile de ricin polyoxyéthyléné, stéarate, de préférence de calcium, de magnésium, d'aluminium ou de zinc, stéarylfumarate, de préférence de sodium, béhénate de glycérol ; pris à eux seuls ou en mélange entre eux;

⇒ <u>Famille C :</u>

♦ 1C

~ les dérivés non hydrosolubles de la cellulose, l'éthylcellulose et/ou l'acétate de cellulose, étant particulièrement préféré(s),
~ les dérivés acryliques,
~ les polyvinylacétates,
~ et leurs mélanges.

♦ 2C

~ les dérivés hydrosolubles de la cellulose,
~ les polyacrylamides,
~ les poly-N-vinylamides,
~ les poly-N-vinyl-lactames,
~ les alcools polyvinyliques (APV),
~ les polyoxyéthylènes (POE),
~ les polyvinylpyrrolidones (PVP) (ces dernières étant préférées),
~ et leurs mélanges ;

♦ 3C

~ le glycérol et ses esters, de préférence dans le sous-groupe suivant: glycérides acétylés, glycérolmonostéarate, glycéryltriacétate, glycéroltributyrate,
~ les phtalates, de préférence dans le sous-groupe suivant : dibutylphtalate, diéthylphtalate, diméthylphtalate, dioctylphtalate,
~ les citrates, de préférence dans le sous-groupe suivant : acétyl-tributylcitrate, acétyltriéthylcitrate, tributylcitrate, triéthylcitrate,
~ les sébacates, de préférence dans le sous-groupe suivant : diéthylsébacate, dibutylsébacate,
~ les adipates,
~ les azélates,
~ les benzoates,
~ les huiles végétales,
~ les fumarates de préférence le diéthylfumarate,
~ les malates, de préférence le diéthylmalate,
~ les oxalates, de préférence le diéthyloxalate,
~ les succinates; de préférence le dibutylsuccinate,
~ les butyrates,
~ les esters de l'alcool cétylique,
~ les malonates, de préférence le diéthylmalonate,
~ l'huile de ricin (celle-ci étant particulièrement préférée),
~ et leurs mélanges;

♦ 4C

~ les sels alcalins ou alcalinoterreux des acides gras, l'acide stéarique et/ou oléique étant préféré(s),
- les huiles polyoxyéthylénées de préférence l'huile de ricin hydrogénée polyoxyéthylénée,
~ les copolymères polyoxyéthylène-polyoxypropylène,
~ les esters de sorbitan polyoxyéthylénés,
~ les dérivés de l'huile de ricin polyoxyéthylénés,
~ les stéarates, de préférence de calcium, de magnésium, d'aluminium ou de zinc,
~ les stéarylfumarates, de préférence de sodium,
~ le béhénate de glycérol,
~ et leurs mélanges.

3. Suspension selon la revendication 1 ou 2, **caractérisée en ce que** la pellicule d'enrobage est constituée par une seule couche.

4. Suspension selon la revendication 1, **caractérisée en ce que** la phase liquide est au moins en partie, de préférence totalement, saturée en principe(s) actif(s) consécutivement à l'incorporation des microcapsules dans cette phase liquide.

5. Suspension selon la revendication 4, **caractérisée en ce que** la saturation en principe(s) actif(s) s'opère au moyen du (ou des) principe(s) actif(s) contenu(s) dans les microcapsules.

6. Suspension selon la revendication 1, **caractérisée en ce que** la phase liquide est au moins en partie, de préférence totalement, saturée en principe(s) actif(s) à l'aide de principe(s) actif(s) non encapsulé(s), avant l'incorporation des microcapsules dans cette phase liquide.

7. Suspension selon l'une quelconque des revendications 1 à 6, **caractérisée en ce que** les microcapsules ont une granulométrie inférieure ou égale à 1000 microns, de préférence comprise entre 200 et 800 microns, et, plus préférentiellement encore, entre 200 et 600 microns.

8. Suspension selon l'une quelconque des revendications 1 à 7, **caractérisée en ce que** la pellicule d'enrobage représente de 1 à 50 % en poids, de préférence de 5 à 40 % en poids, de la masse totale des microcapsules enrobées.

9. Suspension selon la revendication 8, **caractérisée par** un profil de libération in vitro réalisé à l'aide d'un appareil de type II, selon la pharmacopée européenne 3ème édition, dans un milieu tampon phosphate pH 6,8 et à une température de 37°C, tel que :

► la proportion (%) de principe(s) actif(s) libéré(s) durant les 15 premières minutes du test de dissolution est telle que:

$$PI \leq 15$$

de préférence

$$PI \leq 5,$$

► la libération du (ou des) principe(s) actif(s) restant(s) s'effectue sur une durée telle que le temps de libération de 50 % poids de PA ($t_{1/2}$) se définit comme suit (en heures) :

$$0,5 \leq t_{1/2} \leq 30$$

de préférence

$$0,5 \leq t_{1/2} \leq 20.$$

**10.** Suspension selon l'une quelconque des revendications 1 à 9, **caractérisée en ce que** :

- le profil de libération in vitro initial Pfi, réalisé juste après la mise en suspension des microcapsules dans la phase solvant (de préférence aqueuse), à l'aide d'un appareil de type II, selon la pharmacopée européenne 3ème édition, dans un milieu tampon phosphate pH 6,8, à une température de 37°C,
- et le profil de libération in vitro Pf10, réalisé 10 jours après la mise en suspension des microcapsules dans la phase solvant (de préférence aqueuse), à l'aide d'un appareil de type II, selon la pharmacopée européenne 3ème édition, dans un milieu tampon phosphate pH 6,8, à une température de 37°C,

sont similaires.

**11.** Suspension selon l'une quelconque des revendications 1 à 10, **caractérisée en ce que** son pH est indifféremment acide ou neutre.

**12.** Suspension selon l'une quelconque des revendications 1 à 11, **caractérisée en ce qu'**elle comprend au moins un agent modificateur de la rhéologie.

**13.** Suspension selon l'une quelconque des revendications 1 à 12, **caractérisée en ce qu'**elle comprend au moins un agent modificateur de la solubilité du (ou des) principe(s) actif(s) dans la phase liquide solvant (de préférence aqueuse).

**14.** Suspension selon l'une quelconque des revendications 1 à 13, **caractérisée en ce qu'**elle comprend au moins un additif choisi dans le groupe comprenant :

les tensioactifs, les colorants, les agents dispersants, les conservateurs, les agents de sapidité, les arômes, les édulcorants, les anti-oxydants et leurs mélanges.

**15.** Suspension selon l'une quelconque des revendications 1 à 14, **caractérisée en ce que** le (ou les) principe(s) actif(s) appartient (appartiennent) à au moins l'une des familles de substances actives suivantes :

antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

**16.** Suspension selon la revendication 15, **caractérisée en ce que** le PA est choisi parmi les composés suivants :

pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, périndopril, morphine, pentazocine, metformine, paracétamol, oméprazole, métoctopramide, aténolol, salbutamol morphine, vérapamil, erythromycine, caféine, furosémide, céphalosporines, montelukast, valacyclovir, sels d'acide ascorbique, diazépam, théophilline, ciprofloxacine, vancomycine, aminoglycosides, pénicillines (à l'exception de l'amoxicilline) et leurs mélanges.

17. Médicament **caractérisé en ce qu'**il comprend de la suspension selon l'une quelconque des revendications 1 à 16.

18. Médicament **caractérisé en ce qu'**il comprend un nécessaire de préparation de la suspension selon l'une quelconque des revendications 1 à 16, lequel nécessaire comporte:

- des microcapsules sous forme sensiblement sèche contenant le (ou les) principe(s) actif(s) pour permettre la saturation de la phase liquide en principe(s) actif(s), une fois réalisée la mise en présence des deux phases solide et liquide;
- et/ou un mélange de microcapsules sous forme sensiblement sèche comprenant la dose en principe(s) actif(s) juste nécessaire pour la libération modifiée ainsi qu'une dose nécessaire et suffisante de principe(s) actif(s) non enrobé(s) à libération immédiate, pour permettre la saturation de la phase liquide en principe(s) actif(s), une fois réalisée la mise en présence de la dose de saturation de principe(s) actif(s) et de la phase liquide;
- et la phase liquide et/ou au moins une partie des ingrédients utiles à sa préparation et/ou le protocole de préparation de la suspension.

**Claims**

1. Suspension of microcapsules in an aqueous liquid phase that allows the modified release of at least one active principle (excluding amoxicillin) and is intended for oral administration, **characterized in that**:

■ it comprises a plurality of microcapsules each consisting of a core containing at least one active principle (excluding amoxicillin) and of a film coating that:

    • is applied to the core,
    • controls the modified release of the active principle(s),
    • and has a composition corresponding to one of the following families A or C: or

        ⇒ Family A

            ♦ 1A -at least one film-forming polymer (P1) insoluble in the gastrointestinal tract fluids, present in an amount of 50 to 90%, preferably of 50 to 80% by dry weight based on the total weight of the coating composition, and consisting of at least one water-insoluble cellulose derivative;
            ♦ 2A -at least one nitrogen-containing polymer (P2) present in an amount of 2 to 25%, preferably of 5 to 15% by dry weight based on the total weight of the coating composition, and consisting of at least one polyacrylamide and/or poly-N-vinylamide and/or poly-N-vinyllactam;
            ♦ 3A -at least one plasticizer present in an amount of 2 to 20%, preferably of 4 to 15% by dry weight based on the total weight of the coating composition, and consisting of at least one of the following compounds: glycerol esters, phthalates, citrates, sebacates, cetyl alcohol esters and castor oil;
            ♦ 4A -at least one surfactant and/or lubricant present in an amount of 2 to 20%, preferably of 4 to 15% by dry weight based on the total weight of the coating composition, and selected among anionic surfactants and/or non-ionic surfactants and/or lubricants, it being possible for said agent to comprise only one, or a mixture, of the above-mentioned products;

        ⇒ Family C

            ♦ 1C -at least one film-forming polymer insoluble in the gastrointestinal tract fluids;
            ♦ 2C -at least one water-soluble polymer;

♦ 3C -at least one plasticizer;
♦ 4C -and optionally at least one surfactant/lubricant, preferably selected among the following group of products:

~ anionic surfactants;
~ and/or non-ionic surfactants;

■ it comprises:

- 30 to 95% by weight, preferably 60 to 85% by weight of liquid phase (advantageously water),
- 5 to 70% by weight, preferably 15 to 40% by weight of microcapsules;

■ the amount of solvent liquid phase (preferably water) for the active principle(s) is such that the proportion of dissolved active principle(s) originating from the microcapsules is less than or equal to 15%, preferably to 5% by weight based on the total weight of the active principle(s) contained in the microcapsules.

■ and the liquid phase is saturated or becomes saturated with active principle(s) on contact with the microcapsules.

2. Suspension according to claim 1, **characterized in that** the families A and C from which the constituents of the coating composition are selected are as follows:

⇒ Family A

♦ 1A- ethyl cellulose and/or cellulose acetate;
♦ 2A- polyacrylamide and/or polyvinylpyrrolidone;
♦ 3A- castor oil;
♦ 4A- alkali metal or alkaline earth metal salts of fatty acids, stearic and/or oleic acid being preferred, a polyethoxylated sorbitan ester, a polyethoxylated castor oil derivative, a stearate, preferably calcium, magnesium, aluminium or zinc stearate, a stearylfumarate, preferably sodium stearylfumarate, or glycerol behenate, taken individually or in a mixture with one another;

⇒ Family C

♦ 1C

~ water-insoluble cellulose derivatives, ethyl cellulose and/or cellulose acetate being particularly preferred,
~ acrylic derivatives,
- polyvinyl acetates,
~ and mixtures thereof;

♦ 2C

- water-soluble cellulose derivatives,
~ polyacrylamides,
- poly-N-vinylamides,
- poly-N-vinyllactams,
- polyvinyl alcohols (PVA),
~ polyoxyethylenes (POE),
~ polyvinylpyrrolidones (PVP) (the latter being prefemed),
~ and mixtures thereof;

♦ 3C

~ glycerol and its esters, preferably from the following subgroup: acetylated glycerides, glycerol monostearate, glyceryl triacetate and glycerol tributyrate,
~ phthalates, preferably from the following subgroup: dibutyl phthalate, diethyl phthalate, dimethyl phthalate and dioctyl phthalate,

~ citrates, preferably from the following subgroup: acetyltributyl citrate, acetyltriethyl citrate, tributyl citrate and triethyl citrate,

~ sebacates, preferably from the following subgroup: diethyl sebacate and dibutyl sebacate,

~ adipates,

~ azelates,

~ benzoates,

~ vegetable oils,

~ fumarates, preferably diethyl fumarate,

~ malates, preferably diethyl malate,

~ oxalates, preferably diethyl oxalate,

- succinates, preferably dibutyl succinate,

- butyrates,

~ cetyl alcohol esters,

~ malonates, preferably diethyl malonate,

~ castor oil (this being particularly preferred),

~ and mixtures thereof;

♦ 4C

~ alkali metal or alkaline earth metal salts of fatty acids, stearic and/or oleic acid being preferred,

~ polyethoxylated oils, preferably polyethoxylated hydrogenated castor oil,

~ polyoxyethylene/polyoxypropylene copolymers,

~ polyethoxylated sorbitan esters,

~ polyethoxylated castor oil derivatives,

~ stearates, preferably calcium, magnesium, aluminium or zinc stearate,

~ stearylfumarates, preferably sodium stearylfumarate,

~ glycerol behenate,

~ and mixtures thereof.

**3.** Suspension according to claim 1 or 2, **characterized in that** the film coating consists of a single layer.

**4.** Suspension according to claim 1, **characterized in that** the liquid phase is at least partially, preferably totally saturated with active principle(s) following the incorporation of the microcapsules into this liquid phase.

**5.** Suspension according to claim 4, **characterized in that** the saturation with active principle(s) is obtained by means of the active principle(s) contained in the microcapsules.

**6.** Suspension according to claim 1, **characterized in that** the liquid phase is at least partially, preferably totally, saturated with active principle(s) by means of non-encapsulated active principle(s) before the incorporation of the microcapsules into this liquid phase.

**7.** Suspension according to any one of claims 1 to 6, **characterized in that** the microcapsules have a particle size less than or equal to 1000 microns, preferably between 200 and 800 microns and particularly preferably between 200 and 600 microns.

**8.** Suspension according to any one of claims 1 to 7, **characterized in that** the film coating represents from 1 to 50% by weight, preferably 5 to 40%, of the total weight of the coated microcapsules.

**9.** Suspension according to claim 8, **characterized by** an in vitro release profile obtained using a type II apparatus according to the European Pharmacopoeia 3rd edition, in a phosphate buffer medium of pH 6.8 and at a temperature of 37°C, such that:

► the proportion (%) P1 of active principle(s) released during the first 15 minutes of the dissolution test is such that:

$$P1 \leq 15$$

preferably

$$PI \leq 5,$$

► the remaining active principle(s) is (are) released over a period such that the release time of 50% by weight of active principle(s) ($t_{1/2}$) is defined as follows (in hours):

$$0.5 \leq t_{1/2} \leq 30$$

preferably

$$0.5 \leq t_{1/2} \leq 20.$$

10. Suspension according to any one of claims 1 to 9, **characterized in that**:

- the initial in vitro release profile Pfi obtained just after suspension of the microcapsules in the solvent (preferably aqueous) phase, using a type II apparatus according to the European Pharmacopoeia 3rd edition, in a phosphate buffer medium of pH 6.8, at a temperature of 37°C,
- and the in vitro release $Pf_{10}$ profile obtained 10 days after suspension of the microcapsules in the solvent (preferably aqueous) phase, using a type II apparatus according to the European Pharmacopoeia 3rd edition, in a phosphate buffer medium of pH 6.8, at a temperature of 37°C,

are similar.

11. Suspension according to any one of claims 1 to 10, **characterized in that** its pH is arbitrarily acidic or neutral.

12. Suspension according to any one of claims 1 to 11, **characterized in that** it comprises at least one rheology modifier.

13. Suspension according to any one of claims 1 to 12, **characterized in that** it comprises at least one agent for modifying the solubility of the active principle(s) in the solvent liquid (preferably aqueous) phase.

14. Suspension according to any one of claims 1 to 13, **characterized in that** it contains at least one additive selected from the group comprising:

surfactants, colorants, dispersants, preservatives, taste improvers, flavourings, sweeteners, antioxidants and mixtures thereof.

15. Suspension according to any one of claims 1 to 14, **characterized in that** the active principle(s) belong(s) to at least one of the following families of active substances: antiulcer drugs, antidiabetics, anticoagulants, antithrombics, hypolipidaemics, antiarrhythmics, vasodilators, antiangina drugs, antihypertensives, vasoprotectors, fertility promoters, labour inducers and inhibitors, contraceptives, antibiotics, antifungals, antivirals, anticancer drugs, antiinflammatories, analgesics, antiepileptics, antiparkinsonism drugs, neuroleptics, hypnotics, anxiolytics, psychostimulants, antimigraine drugs, antidepressants, antitussives, antihistamines and antiallergics.

16. Suspension according to claim 15, **characterized in that** the active principle is selected among the following compounds: pentoxifylline, prazosin, aciclovir, nifedipine, diltiazem, naproxen, ibuprofen, flurbiprofen, ketoprofen, fenoprofen, indomethacin, diclofenac, fentiazac, oestradiol valerate, metoprolol, sulpiride, captopril, cimetidine, zidovudine, nicardipine, terfenadine, atenolol, salbutamol, carbamazepine, ranitidine, enalapril, simvastatin, fluoxetine, alprazolam, famotidine, ganciclovir, famciclovir, spironolactone, 5-asa, quinidine, perindopril, morphine, pentazocine, metformin, paracetamol, omeprazole, metoclopramide, atenolol, salbutamol morphine, verapamil, erythromycin, caffeine, furosemide, cephalosporins, montelukast, valaciclovir, ascorbic acid salts, diazepam, theophylline, ciprofloxacin, vancomycin, aminoglycosides, penicillins (except for amoxicillin) and mixtures thereof.

**17.** Drug, **characterized in that** it comprises a suspension according to any one of claims 1 to 16.

**18.** Drug, **characterized in that** it comprises a kit for preparing the suspension according to any one of claims 1 to 16, said kit containing:

- microcapsules in substantially dry form containing the active principle(s) for saturating the liquid phase with active principle(s) once the two solid and liquid phases have been brought into contact;
- and/or a mixture of microcapsules in substantially dry form containing the active principle(s) in the dose that is just necessary for modified release, as well as with immediate-release uncoated active principle(s) in a necessary and sufficient dose to saturate the liquid phase with active principle(s) once the saturation dose of active principle(s) and the liquid phase have been brought into contact;
- and the liquid phase and/or at least part of the ingredients useful for its preparation, and/or the protocol for preparation of the suspension

**Patentansprüche**

**1.** Mikrokapselsuspension in flüssiger wässriger Phase zur modifizierten Freisetzung mindestens eines Wirkprinzips, ausgenommen Amoxicillin, zur oralen Verabreichung, **dadurch gekennzeichnet, dass**:

■ sie eine Vielzahl von Mikrokapseln aus jeweils einem Kern mit mindestens einem Wirkprinzip (ausgenommen Amoxicillin) und einem Umhüllungsfilm umfasst, der:

• auf dem Kern aufgebracht vorliegt,
• die modifizierte Freisetzung des oder der Wirkprinzipien steuert,
• und eine Zusammensetzung aufweist, die einer der folgenden Familien A oder C entspricht:

⇒ Familie <u>A</u>:

♦ 1A - mindestens ein filmbildendes Polymer (P1), unlöslich in den Flüssigkeiten des Magen-Darm-Trakts, vorhanden mit 50 bis 90 Gew.% und vorzugsweise mit 50 bis 80 Gew.%, bezogen auf die trockene Gesamtmasse der Umhüllungszusammensetzung, aus mindestens einem nicht-wasserlöslichen Cellulosederivat;
♦ 2A - mindestens ein stickstoffhaltiges Polymer (P2), vorhanden mit 2 bis 25 Gew.% und vorzugsweise mit 5 bis 15 Gew.%, bezogen auf die trockene Gesamtmasse der Umhüllungszusammensetzung, aus mindestens einem Polyacrylamid und/oder einem Poly-N-vinylamid und/oder einem Poly-N-vinyllactam;
♦ 3A - mindestens ein Plastifiziermittel, vorhanden mit 2 bis 20 Gew.% und vorzugsweise mit 4 bis 15 Gew.%, bezogen auf die trockene Gesamtmasse der Umhüllungszusammensetzung, aus mindestens einer der folgenden Verbindungen: Glycerylestern, Phthalaten, Zitraten, Sebacaten, Estern von Cetylalkohol, Rizinusöl;
♦ 4A - mindestens ein oberflächenaktives und/oder Gleitmittel vorhanden mit 2 bis 20 Gew.% und vorzugsweise mit 4 bis 15 Gew.%, bezogen auf die trockene Gesamtmasse der Umhüllungszusammensetzung, ausgewählt aus anionischen und/oder nicht-ionischen oberflächenaktiven Mitteln und/oder aus Gleitmitteln; wobei das genannte Mittel eines oder eine Mischung aus den oben genannten Produkten umfassen kann;

⇒ <u>Familie C</u>:

♦ 1C - mindestens ein filmbildendes Polymer, unlöslich in den Flüssigkeiten des Magen-Darm-Trakts;
♦ 2C - mindestens ein wasserlösliches Polymer;
♦ 3C - mindestens ein Plastifiziermittel;
♦ 4C - und gegebenenfalls mindestens ein oberflächenaktives Mittel/Gleitmittel, vorzugsweise ausgewählt aus der Gruppe folgender Produkte:

~ anionischen oberflächenaktiven Mitteln,
~ und/oder nicht-ionischen oberflächenaktiven Mitteln;

■ dass sie umfasst:

- 30 bis 95 Gew.% und bevorzugt 60 bis 85 Gew.% flüssige Phase (in vorteilhafter Weise Wasser),
- 5 bis 70 Gew.% und bevorzugt 15 bis 40 Gew.% Mikrokapseln;

■ dass die Menge der flüssigen Lösephase (bevorzugt aus Wasser) des oder der Wirkprinzipien so bemessen ist, dass der Mengenanteil des oder der gelösten Wirkprinzipien, welcher aus den Mikrokapseln hervorgeht, weniger als oder gleich 15 Gew.% und bevorzugt 5 Gew.% beträgt, bezogen auf die Gesamtmasse des oder der in den Mikrokapseln enthaltenen Wirkprinzipien;
■ und dass die flüssige Phase bei Kontakt mit den Mikrokapseln mit dem oder den Wirkprinzipien gesättigt ist oder sich damit sättigt.

2. Suspension gemäß Anspruch 1, **dadurch gekennzeichnet, dass** die Familien A, und C aus denen die Bestandteile der Umhüllungszusammensetzung ausgewählt sind, die folgenden sind:

⇒ Familie A:

♦ 1A - Ethylcellulose und/oder Celloseacetat;
♦ 2A - Polyacrylamid und/oder Polyvinylpyrrolidon;
♦ 3A - Rizinusöl;
♦ 4A - Alkali- oder Erdalkalisalze von Fettsäuren, bevorzugt von Stearin- und/oder Ölsäure, polyoxyethylierter Sorbitanester, polyoxyethyliertes Rizinusölderivat, Stearat, bevorzugt von Calcium, Magnesium, Aluminium oder Zink, Stearylfumarat, bevorzugt von Natrium, Glycerylbehenat; jeweils einzeln oder als Mischung aus diesen;

⇒ Familie C:

♦ 1C:

~ nicht-wasserlösliche Cellulosederivate, bevorzugt Ethylcellulose und/oder Celluloseacetat,
~ Acrylderivate,
~ Polyvinylacetate,
~ und deren Mischungen;

♦ 2C:

~ wasserlösliche Cellulosederivate,
~ Polyacrylamide,
~ Poly-N-vinylamide,
~ Poly-N-vinyllactame,
~ Polyvinylalkohole (PVA),
~ Polyoxyethylene (POE),
~ Polyvinylpyrrolidone (PVP) (wobei diese letzteren bevorzugt sind),
~ und deren Mischungen;

♦ 3C:

~ Glycerin und seine Ester, bevorzugt aus der folgenden Untergruppe: acetylierte Glyceride, Glycerylmonostearat, Glyceryltriacetat, Glyceryltributyrat,
~ Phthalate, bevorzugt aus der folgenden Untergruppe: Dibutylphthalat, Diethylphthalat, Dimethylphthalat, Dioctylphthalat,
~ Zitrate, bevorzugt aus der folgenden Untergruppe: Acetyltributylzitrat, Acetyltriethylzitrat, Tributylzitrat, Triethylzitrat,
~ Sebacate, bevorzugt aus der folgenden Untergruppe: Diethylsebacat, Dibutylsebacat,
~ Adipate,
~ Azelate,
~ Benzoate,
~ Pflanzenöle,

~ Fumarate, bevorzugt Diethylfumarat,
~ Malate, bevorzugt Diethylmalat,
~ Oxalate, bevorzugt Diethyloxalat,
~ Succinate, bevorzugt Dibutylsuccinat,
~ Butyrate,
~ Ester von Cetylalkohol,
~ Malonate, bevorzugt Diethylmalonat,
~ Rizinusöl (wobei dieses besonders bevorzugt ist),
~ und deren Mischungen;

   ♦ 4C:

~ Alkali- oder Erdalkalisalze von Fettsäuren, bevorzugt von Stearin- und/oder Ölsäure,
~ polyoxyethylierte Öle, bevorzugt hydriertes polyoxyethyliertes Rizinusöl,
~ Polyoxyethylen-Polyoxypropylen-Copolymere,
~ polyoxyethylierte Sorbitanester,
~ polyoxyethylierte Rizinusölderivate,
~ Stearate, bevorzugt von Calcium, Magnesium, Aluminium oder Zink,
~ Stearylfumarate, bevorzugt von Natrium,
~ Glycerylbehenat,
~ und deren Mischungen.

3. Suspension gemäß Anspruch 1 oder 2, **dadurch gekennzeichnet, dass** der Umhüllungsfilm aus einer einzigen Schicht zusammengesetzt ist.

4. Suspension gemäß Anspruch 1, **dadurch gekennzeichnet, dass** die wässrige Phase zumindest teilweise und bevorzugt gänzlich mit Wirkprinzip(ien) nacheinander bei Einbringung der Mikrokapseln in diese flüssige Phase gesättigt ist.

5. Suspension gemäß Anspruch 4, **dadurch gekennzeichnet, dass** die Sättigung mit Wirkprinzip(ien) des oder der in den Mikrokapseln enthaltenen Wirkprinzipien erfolgt.

6. Suspension gemäß Anspruch 1, **dadurch gekennzeichnet, dass** die flüssige Phase zumindest teilweise und bevorzugt gänzlich mit Wirkprinzip(ien) mittels nicht verkapseltem(n) Wirkprinzip(ien) vor Einbringung der Mikrokapseln in diese flüssige Phase gesättigt ist.

7. Suspension gemäß einem der Ansprüche 1 bis 6, **dadurch gekennzeichnet, dass** die Mikrokapseln eine Korngröße von weniger als oder gleich 1.000 $\mu$m , bevorzugt von 200 bis 800 und noch bevorzugter von 200 bis 600 $\mu$m aufweisen.

8. Suspension gemäß einem der Ansprüche 1 bis 7, **dadurch gekennzeichnet, dass** der Umhüllungsfilm 1 bis 50 Gew.% und bevorzugt 5 bis 40 Gew.% der Gesamtmasse der umhüllten Mikrokapseln ausmacht.

9. Suspension gemäß Anspruch 8, **gekennzeichnet durch** ein in vitro-Freisetzprofil, durchgeführt mittels einer Typ II-Vorrichtung gemäß dritter Ausgabe der europäischen Pharmacopea in einem Phosphatpuffermilieu von pH = 6,8 bei einer Temperatur von 37°C, wobei gilt:

   ► der Mengenanteil (%) P1 des oder der während der 15 ersten Minuten des Auflösungstests freigesetzten Wirkprinzip(ien) so bemessen ist, dass gilt:

$$P1 \leq 15$$

   bevorzugt

$$Pl \leq 5,$$

► die Freisetzung des (der) restlichen Wirkprinzip(ien) erfolgt über eine solche Dauer, dass die Freisetzzeit von 50 Gew.% Wirkprinzip(ien) ($t_{1/2}$) wie folgt (in Stunden) definiert ist:

$$0,5 \rightarrow \leq t_{1/2} \leq 30$$

bevorzugt

$$0,5 \rightarrow \leq t_{1/2} \leq 20.$$

**10.** Suspension gemäß einem der Ansprüche 1 bis 9, **dadurch gekennzeichnet, dass**:

- das anfängliche in vitro-Freisetzprofil Pfi, durchgeführt kurz nach Suspendierung der Mikrokapseln in der (bevorzugt wässrigen) Lösephase mittels einer Typ II-Vorrichtung gemäß dritter Ausgabe der europäischen Pharmacopea in einem Phosphatpuffermilieu von pH = 6,8 bei einer Temperatur von 37°C,
- und das in vitro-Freisetzprofil $Pf_{10}$, durchgeführt 10 Tage nach der Suspendierung der Mikrokapseln in der (bevorzugt wässrigen) Lösephase mittels der Typ II-Vorrichtung gemäß dritter Ausgabe der europäischen Pharmacopea in einem Phosphatpuffermilieu von pH = 6,8 bei einer Temperatur von 37°C,

ähnlich sind.

**11.** Suspension gemäß einem der Ansprüche 1 bis 10, **dadurch gekennzeichnet, dass** ihr pH-Wert unterschiedslos sauer oder neutral ist.

**12.** Suspension gemäß einem der Ansprüche 1 bis 11, **dadurch gekennzeichnet, dass** sie mindestens ein Rheologiemodifiziermittel umfasst .

**13.** Suspension gemäß einem der Ansprüche 1 bis 12, **dadurch gekennzeichnet, dass** sie mindestens ein Modifiziermittel der Löslichkeit des (oder der) Wirkprinzip(ien) der (bevorzugt wässrigen) flüssigen Lösephase umfasst.

**14.** Suspension gemäß einem der Ansprüche 1 bis 13, **dadurch gekennzeichnet, dass** sie mindestens ein Additiv umfasst, ausgewählt aus der Gruppe aus:

oberflächenaktiven Mitteln, Farbstoffen, Dispergiermitteln, Konservierungsstoffen, Geschmacksstoffen, Aromen, Süßungsmittel, Antioxidanzien und aus deren Mischungen.

**15.** Suspension gemäß einem der Ansprüche 1 bis 14, **dadurch gekennzeichnet, dass** das (oder die) Wirkprinzip(ien) zu mindestens einer der Familien der folgenden Wirksubstanzen gehören:

Antimagengeschwürmittel, Antidiabetika, Antikoagulanzien, Antithrombika, Hypolipämiemittel, Antiarythmika, Vasodilatatoren, Antianginamittel, Antihochdruckmittel, Vasoprotektoren, Fruchtbarkeitsförderungsmittel, Induktoren und Inhibitoren der Gebärmutterarbeit, Verhütungsmittel, Antibiotika, Antifungika, antivirale Mittel, Antikrebsmittel, Antientzündungsmittel, Analgetika, Antiepilleptika, Antiparkisonmittel, Neuroleptika, Hypnotika, Anxiolytika, Psychostimulanzien, Antimigränemittel, Antidepressiva, Antitussiva, Antihistaminika oder Antiallergika.

**16.** Suspension gemäß Anspruch 15, **dadurch gekennzeichnet, dass** das Wirkprinzip aus den folgenden Verbindungen ausgewählt ist: Pentoxifyllin, Prazosin, Acyclovir, Nifedipin, Diltiazem, Naproxen, Ibuprofen, Flurbiprofen, Ketoprofen, Fenoprofen, Indomethacin, Diclofenac, Fentiazac, Östradiolvalerat, Metoprolol, Sulpirid, Captopril, Cimetidin, Zidovudin, Nicardipin, Terfenadin, Atenolol, Salbutamol, Carbamazepin, Ranitidin, Enalapril, Simvastatin, Fluoxetin, Alprazolam, Famotidin, Ganciclovir, Famciclovir, Spironolacton, 5-Asa, Chinidin, Perindopril, Morphin, Pentazocin, Metformin, Paracetamol, Omeprazol, Metoclopramid, Atenolol, Salbutamol Morphin, Verapamil, Erythromycin, Coffein, Furosemid, Cephalosporinen, Montelukast, Valacyclovir, Ascorbinsäuresalzen, Diazepam, Theophillin, Ciprofloxacin, Vancomycin, Aminoglycosiden, Penicillinen (ausgenommen Amoxicillin) und aus deren Mischungen.

**17.** Medikament, **dadurch gekennzeichnet, dass** es die Suspension gemäß einem der Ansprüche 1 bis 16 umfasst.

**18.** Medikament, **dadurch gekennzeichnet, dass** es einen notwendigen Zubereitungsbestandteil der Suspension gemäß einem der Ansprüche 1 bis 16 umfasst, wobei der notwendige Bestandteil umfasst:

- Mikrokapseln in im Wesentlichen trockener Form, enthaltend das (oder die) Wirkprinzip(ien) zur Sättigung der flüssigen Phase mit Wirkprinzip(ien) beim Zusammenbringen der beiden festen und flüssigen Phasen;
- und/oder eine Mischung von Mikrokapseln in im Wesentlichen trockener Form, umfassend die zur modifizierten Freisetzung gerade notwendige Dosis an Wirkprinzip(ien) sowie eine notwendige und hinreichende Dosis an nicht umhülltem(en) Wirkprinzip(ien) bei unmittelbarer Freisetzung zur Sättigung der flüssigen Phase mit Wirkprinzip(ien) beim Zusammenbringen der Sättigungsdosis an Wirkprinzip(ien) und der flüssigen Phase;
- und die flüssige Phase und/oder mindestens einen Teil der Bestandteile, die zur Zubereitung und oder dem Zubereitungsprotokoll der Suspension verwendet werden.

**FIG.1**

**FIG.2**

**FIG. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0709087 B
- FR 2634377 A
- US 4999189 B
- US 5186930 B
- WO A8707833 A
- US 4902513 B
- EP 0601508 A
- WO A9601628 A
- EP 0853693811 A
- EP 0273890 A
- EP 0953359 B

**Littérature non-brevet citée dans la description**

- pharmacopée européenne
- The European Agency for the Evaluation of Medicinal Products (EMEA)- Human Medicines Evaluation Unit. *Committee for proprietary medicinal products (CPMP) - London,* 29 Juillet 1999
- Handbook of pharmaceutical excipients. Am. Pharmaceutical Association, 2000
- **Arthur H KIBBE.** Handbook of pharmaceutical excipients. Am. Pharmaceutical Association, 2000
- **C. DUVERNEY ; J.P. BENOIT.** *L'actualité chimique,* Décembre 1986
- pharmacopée Européenne. vol. 900
- pharmacopée Européenne. vol. 1000